# EUROPÄISCHE PATENTSCHRIFT

(11) **EP 1 299 191 B1**
(45) Veröffentlichungstag und Bekanntmachung des Hinweises auf die Patenterteilung: **19.09.2007**
(21) Anmeldenummer: 01947441.0
(22) Anmeldetag: 04.07.2001
(51) Int. Cl.: B01J 37/16, B01J 35/10, B01J 23/755, C07C 209/36

(54) **METALLISCHE NICKELHYDRIERKATALYSATOREN, DEREN HERSTELLUNG UND DEREN VERWENDUNG**
METALLIC NICKEL HYDROGENATION CATALYSTS, PRODUCTION AND USE THEREOF
CATALYSEURS DE NICKEL METALLIQUES D'HYDROGENATION, LEUR PRODUCTION ET LEUR UTILISATION

(30) Priorität: 04.07.2000 DE 10032303
(43) Veröffentlichungstag der Anmeldung: 09.04.2003
(73) Patentinhaber: Leibniz-Institut für Neue Materialien gemeinnützige GmbH, 66123 Saarbrücken (DE)
(72) Erfinder: VANOPPEN, Dominic, 67105 Schifferstadt (DE); VEITH, Michael, 66386 St. Ingbert (DE); VALTCHEV, Kroum, 66113 Saarbrücken (DE)
(86) Internationale Anmeldenummer: PCT/EP2001/007650
(87) Internationale Veröffentlichungsnummer: WO 2002/002233

(56) Entgegenhaltungen:
- EP-A- 0 168 096
- WO-A-00/51727
- HESSE J ET AL: "Different susceptibilities of nanosized single-domain particles derived from magnetisation measurements" JOURNAL OF MAGNETISM AND MAGNETIC MATERIALS, ELSEVIER SCIENCE PUBLISHERS, AMSTERDAM, NL, Bd. 212, Nr. 1-2, März 2000 (2000-03), Seiten 153-167, XP004191479 ISSN: 0304-8853
- VEITH M ET AL: "Molecular precursor approach to nano-scaled ceramics and metal/metal oxide composites" NANOSTRUCTURED MATERIALS, ELSEVIER, NEW YORK, NY, US, Bd. 12, Nr. 1-4, 1999, Seiten 191-194, XP004176970 ISSN: 0965-9773 in der Anmeldung erwähnt

## Beschreibung

Die Erfindung betrifft metallische Nickel-Hydrierkatalysatoren, die insbesondere zur Hydrierung von aromatischen Nitroverbindungen zu aromatischen Aminen geeignet sind, die jedoch ferner auch für reduktive Aminierungen, Dehydrierungen oder Isomerisierungen eingesetzt werden können.

Bei der Herstellung von Polyurethanen werden häufig aromatische Diisocyanate mit Diolen umgesetzt. Die aromatischen Diisocyanate können dabei durch Nitrierung entsprechender aromatischer Verbindungen, nachfolgende Hydrierung der Nitrogruppen zu Aminogruppen und darauffolgende Umsetzung der Aminogruppen mit Phosgen zu Isocyanatgruppen hergestellt werden. Beispielsweise kann Toluol durch Umsetzung mit Salpetersäure zu Dinitrotoluol nitriert werden, worauf die Nitrogruppen hydriert und in Isocyanatgruppen überführt werden. Bei der Hydrierung sollen die Nitrogruppen in hoher Ausbeute möglichst selektiv hydriert werden, ohne daß es zu einer Hydrierung der aromatischen Kerne kommt.

Als Hydrierkatalysatoren werden häufig Raney-Katalysatoren eingesetzt. Der am leichtesten zugängliche und daher am meisten verwendete Raney-Katalysator ist Raney-Nickel.

Raney-Nickel wird durch Legieren von Nickel mit Aluminium, Silizium, Magnesium oder Zinn und Zersetzung der Legierung nach mechanischer Zerkleinerung mit KOH gewonnen. Dabei wird das katalytisch unwirksame Metall herausgelöst, und ein schwarzer Metallschwamm des katalytisch aktiven Nickels bleibt zurück. Das so erhaltene Raney-Nickel ist jedoch aufgrund der großen freien Metalloberfläche pyrophor, so daß es nur unter Schutzgas oder in inerten Verdünnungsmitteln gehandhabt werden kann. Raney-Nickel wird häufig unmittelbar vor dem Einsatz frisch hergestellt, da beim Aufbewahren unter inerten Verdünnungsmitteln ein starker Aktivitätsverlust eintritt. Ein durch Oxidation desaktivierter Katalysator kann jedoch nicht regeneriert werden.

Es wurde auch versucht, aktive Katalysatoren aus entsprechenden Metallsalzen oder Metallkomplexen durch Aktivierung mit aluminiumorganischen Verbindungen zu erhalten, siehe beispielsweise BE-A-0 737 574. Beispielsweise wird ein Nickelkomplex in der Flüssigphase mit AlH(Ot-Bu)₂ aktiviert.

Anstelle von Raney-Nickel können auch geträgerte Nickelkatalysatoren eingesetzt werden. Zu ihrer Herstellung wird in der Regel der Träger mit einer Nickelverbindung imprägniert oder Träger und Nickelverbindung werden gemeinsam gefällt. Der so erhaltene Katalysator muß vor der Verwendung aktiviert werden, um das Nickeloxid in metallisches Nickel zu überführen. Dabei entsteht ein hochoberflächiges pyrophores Nickel, das passiviert und vor dem Einsatz reaktiviert werden muß.

Gemäß der DD-A-284 371 wird versucht, Nachteile der bekannten Nickelkatalysatoren dadurch zu umgehen, daß eine grobkörniger Nickelkatalysator an der Luft oberflächlich oxidiert wird und damit an der Luft handhabbar wird. Vor dem Einsatz in Hydrierreaktionen wird der Katalysator in eine inerte Flüssigkeit eingetragen und zu einem feinen Pulver vermahlen. Hierbei muß wiederum unter Wasserstoff- oder Inertgasatmosphäre gearbeitet werden.

In Nano Structured Materials, Vol. 12, 1999, Seiten 191 bis 194 ist die Herstellung von Keramiken und Metall/Metalloxid-Composites im Nano-Maßstab aus molekularen Vorläufern beschrieben.

Dabei wird Ni/Al₂O₃ durch Reduktion von NiO mit [H₂Al(O-tert.-Bu)]₂ hergestellt. Die Teilchengröße der Nickelkristallite betrug 50 bis 100 nm. Unter Verwendung von [Ni{Al(O-i-Pr)₄}₂] konnte in einem CVD-Verfahren Ni/Al₂O₃ im Nano-Maßstab hergestellt werden. Die Nickelkristallite weisen jedoch noch verhältnismäßig große Kristallitgrößen im Bereich von 50 bis 100 nm auf.

Die Dissertation von E.W. Fritscher, Universität des Saarlandes, 1997, Seiten 118 bis 140, betrifft dieselben Katalysatorsysteme. Das Ni/Al₂O₃-Material wird bei der Hydrierung von Styrol und Maleinsäure eingesetzt

EP-A 0 168 096 betrifft Hydrierkatalysatoren, die aus Hydriermetall und Siliciumdioxid aufgebaut sind. Als Hydriermetalle kommen Kobalt oder Kupfer in Betracht. Der Katalysator enthält Metall/Metallverbindungs-Aggregate, deren durchschnittliche Teilchengröße zwischen 5 und 50 µm liegt. Die Herstellung erfolgt durch Fällung, Altern, Auftrennen und Reduktion. Dabei wird ein Metallhydroxid oder Metallcarbonat unter Zusatz von Alkali gefällt.

J. Hesse et al., "Different susceptibilities of nanosized single-domain particles derived from magnetisation measurements", Journal of Magnetism and Magnetic Materials, 2000, 212, 1-2, Seiten 157 167 beschreiben die Herstellung von kleinen Nickelteilchen, die in Aluminiumalkoxid dispergiert vorliegen. Dazu wird eine Nickelverbindung mit- bis-(tert.-Butoxyaluminiumdihydrid) umgesetzt. Es wird in Lösung in Toluol gearbeitet. Als Nickel-Precursor wird Nickelacetylacetonat eingesetzt. Die dabei erhaltenen Kristallitgrößen der Nickelteilchen liegen bei 5 bzw. 2,1 nm. Es ist ferner angegeben, dass Nanopartikel von Nickel und anderen ferromagnetischen Materialien für chemische Katalyse interessant sind.

US 4,762,959 betrifft Kobaltkatalysatoren und deren Verwendung zur Methanolumwandlung und für Fischer-Tropsch-Synthesen zur Herstellung von Kohlenwasserstoffen. Der Katalysator enthält 2 bis 25 % Kobalt als Composit mit Titandioxid oder einem Titandioxid enthaltenden Träger. Zusätzlich enthält der Katalysator Zirconium, Hafnium, Cer oder Uran.

GB-A 832 153 betrifft die katalytische Hydrierung von Dinitroderivaten von Toluol. Als Katalysator wird Nickel auf Siliciumdioxid eingesetzt.

Aufgabe der vorliegenden Erfindung ist die Bereitstellung eines Hydrierkatalysators, insbesondere zur Hydrierung aromatischer Nitroverbindungen, der die Nachteile der bekannten Katalysatoren vermeidet und insbesondere an Luft gehandhabt und einfach aktiviert werden kann und nach der Aktivierung eine hohe Aktivität aufweist. Die Aktivierung soll dabei bei niedrigen Temperaturen möglich sein.

Die Aufgabe wird erfindungsgemäß gelöst durch einen Katalysator, herstellbar durch Reduktion eines, auf einem oxidischen Träger, ausgewählt aus TiO₂, ZrO₂ oder Gemischen davon, vorliegenden Ni- Precursors durch Umsetzung mit mindestens einer Verbindung der allgemeinen Formel (I)

HₙM(OR)₃₋ₙ (I)

mit
- M: Ga oder Al,
- R: CR'₃ oder SiR'₃ mit R' C₁₋₂₀-Alkyl,
- n: 1 oder 2
oder Dimeren oder Oligomeren davon.

Vorzugsweise beträgt hierbei die Kristallitgröße des Nickels im fertigen Katalysator 3 bis 100 nm, vorzugsweise 4 bis30 nm, insbesondere 5 bis 15 nm.

Das Nickel kann im Precursor z.B. in der Form eines Oxids, Hydroxids oder gemischten Oxids/Hydroxids oder auch Carbonats oder Salzes vorliegen. Besonders bevorzugt wird NiO, insbesondere mit einer Kristallitgröße im Bereich von 5 bis 15 nm.

In den Verbindungen der allgemeinen Formel (I), Dimeren oder Oligomeren davon ist M vorzugsweise Al. R ist vorzugsweise CR'₃. R' ist vorzugsweise C₁₋₁₂-Alkyl, besonders bevorzugt C₁₋₆-Alkyl, insbesondere C₁₋₃-Alkyl. Es kann dabei Methyl, Ethyl, Propyl oder Isopropyl sein. Speziell ist R' Methyl, so daß R die Bedeutung tert.-Butyl hat. n hat den Wert 1 oder 2, vorzugsweise 2.

Bevorzugt ist die Verbindung der allgemeinen Formel (I) H₂Al(OCR'₃) mit R'C₁₋₃-Alkyl, speziell Methyl, oder ein Dimer davon. Diese Verbindungen sind als Alane, insbesondere tert.-Butgxyalane bekannt und genau charakterisiert. Verfahren zu ihrer Herstellung sind beispielsweise beschrieben in Z. anorg. allg. Chem. 1968, 358, 44 und Chem. Ber. 1996, 129,381-384.

Bei den Dimeren handelt es sich um zyklische Verbindungen, in denen zwei Al-Atome über O-tert.-Butyl-Reste verbrückt sind. Besonders bevorzugt wird erfindungsgemäß derartiges Di-(tert.-Butoxi-aluminiumdihydrid) eingesetzt.

Die Reduktion, insbesondere des Nickeloxids, zu Nickel erfolgt insbesondere durch Umsetzung mit Di-(tert.-Butoxi-aluminiumdihydrid). Die Umsetzung kann dabei in Suspension erfolgen. Vorzugsweise wird jedoch das Oxid und/oder Hydroxid des hydrieraktiven Nickels, insbesondere Nickeloxid, bei der Umsetzung als Feststoff und die Verbindung der allgemeinen Formel. (I) oder das Dimer oder Oligomer davon, insbesondere Di-(tert.-Butoxi-aluminiumdihydrid), gasförmig eingesetzt. Bei der Umsetzung wird das, gegebenenfalls geträgerte, Oxid/Hydroxid des hydrieraktiven Metalls vorzugsweise mechanisch bewegt, um eine gleichmäßige Reduktion zu erreichen.

Der reduzierte Katalysator enthält gewisse Mengen an Al aus dem Reduktionsmittel.

Bei der Gasphasenzersetzung von Di(tert.-Butoxi-aluminiumdihydrid) über Nickeloxid-Pulver entstehen beispielsweise Komposite der Zusammensetzung Ni/NiO/Al₂O₃/(Al) oder Ni/Al₂O₃/(Al) in Abhängigkeit von der Reaktionsdauer. Das Nickeloxid wird dabei durch die Hydridliganden reduziert. Das Aluminiumoxid wird bei der Pyrolyse des Precursors gebildet. In CVD-Verfahren mit Di(tert.-Butoxi-aluminiumdihydrid) als Precursor können auf einem Nickelsubstrat Al/Al₂O₃-Komposit-Schichten entstehen. Es kann neben einer Reduktion von Nickeloxid zu metallischem Nickel zu einer Beschichtung der entstehenden Nickelteilchen mit der Kompositstruktur kommen. Im fertigen Produkt ist jedoch in der Regel wesentlich weniger Aluminium enthalten, als es nach der Stöchiometrie zu erwarten wäre. Auch geringe Aluminium- oder Nickelhydridanteile können vorliegen.

Die Menge an Aluminium, die im Katalysator eingebaut wird, hängt vom gegebenenfalls verwendeten Katalysatorträger ab. Die Variation der Menge kann durch die Bildung relativ flüchtiger Aluminium-oxi-tert.-butoxi-Verbindungen erklärt werden, die unterschiedlich stark an unterschiedliche Träger adsorbieren.

Wie durch Pulverdiffraktometrie gezeigt werden konnte, liegt Nickel in Form von nanokristallinen Phasen vor. Eine mögliche Aluminiumoxidkomponente liegt amorph vor. In der Röntgendiffraktometrie sind die Reflexe des metallischen Aluminiums wenig intensiv und häufig im Untergrund kaum zu erkennen.

Die Ni-Kristallitgröße läßt sich dabei über die Reaktionstemperatur steuern. Vorzugsweise wird die Umsetzung bei Temperaturen im Bereich von 20 bis 600°C durchgerührt, besonders bevorzugt im Bereich von 200 bis 500°C. Bei einer Umsetzungstemperatur von 400 °C wurden beispielsweise deutlich kleinere Ni-Kristallite gebildet als bei 500 °C.

Bei der Umsetzung wird die Verbindung der allgemeinen Formel (I) vorzugsweise in einem Molverhältnis, bezogen auf Oxid des hydrieraktiven Nickels, von 0,1 bis 10, besonders bevorzugt 0,3 bis 3 eingesetzt.

Im nach der Umsetzung erhaltenen Komposit sind Kristallite des hydrieraktiven Nickels feinst verteilt in eine AlOₓ-Matrix eingebettet. Die Komposite besitzen Eigenschaften, die denen von Raney-Nickel ähnlich sind. Sie weisen im Unterschied zu Raney-Nickel jedoch den großen anwendungstechnischen Vorteil auf, an Luft stabil zu sein und trotzdem bei sehr milden Bedingungen (unter Reaktionsbedingungen) wieder reaktiviert werden zu können. Für die Herstellung der Katalysatoren kann ergänzend auf die eingangs zitierte Literatur verwiesen werden.

Beispielsweise kann eine Aktivierung passivierter Katalysatoren bei einer Temperatur im Bereich von 25 bis 250 °C, vorzugsweise 25 bis 120 °C und einem Wasserstoffdruck im Bereich von 1 bis 200 bar, vorzugsweise 1 bis 25 bar erreicht werden. Im Vergleich dazu läßt sich passivierter Raney-Nickel-Katalysator nicht reaktivieren. Ebenso läßt sich ein nach üblichen Verfahren hergestellter Ni/TiO₂-Katalysator, der passiviert wurde, nicht bei 120 °C aktivieren.

Da die Aktivierung der erfindungsgemäßen Katalysatoren unter Hydrierungsbedingungen möglich ist, ist kein gesonderter Aktivierungsschritt notwendig.

Da die erfindungsgemäßen Katalysatoren Aktivitäten aufweisen, die ähnlich den Aktivitäten von Raney-Nickel sind, können sie als "trockene" Raney-Katalysatoren betrachtet werden, die bei Umgebungsbedingungen stabil sind.

Die Erfindung betrifft auch ein Verfahren zur Herstellung der beschriebenen Katalysatoren, bei dem man die angegebenen Verfahrensschritte durchführt.

Zudem betrifft die Erfindung die Verwendung des beschriebenen Katalysators zur Hydrierung, Dehydrierung oder Isomerisierung von, gegebenenfalls substituierten, Kohlenwasserstoffen. Dabei kann es sich um aliphatische, aromatische oder gemischt aliphatisch-aromatische Kohlenwasserstoffe handeln

Der erfindungsgemäße Katalysator wird vorzugsweise zur Hydrierung beispielsweise von C-C-Doppelbindungen, Carboxylgruppen, Nitrilen, Iminen, Carbonsäuren und Estern oder Aromaten eingesetzt. Die Erfindung betrifft auch ein Verfahren zur Herstellung von aromatischen Verbindungen, die Aminogruppen aufweisen, durch Hydrierung entsprechender Nitrogruppen aufweisender aromatischer Verbindungen in Gegenwart eines Katalysators, herstellbar durch Reduktion eines, auf einem oxidischen Träger, ausgewählt aus TiO₂, ZrO₂ oder Gemischen davon vorliegenden Ni- Precursors durch Umsetzung mit mindestens einer Verbindung der allgemeinen Formel (I).

HₙM(OR)₃₋ₙ (I)

mit
- M: Ga oder Al,
- R: CR'₃ oder SiR' C₁₋₂₀-Alkyl
- n: 1 oder 2
oder Dimeren oder Oligomeren davon.

Vorzugsweise ist der Katalysator wie vorstehend beschrieben aufgebaut.

Beispiele zu hydrierender Verbindungen sind Dinitrotoluol, o-, p-Nitrotoluol und Nitrobenzol.

Wenn der erfindungsgemäße Katalysator in der Umsetzung verbraucht ist, kann er nach bekannten Verfahren reaktiviert werden.

Die Erfindung wird nachstehend anhand von Beispielen näher erläutert.

### Beispiel 1

### Darstellung von Bis[(tert.-Butoxy)aluminium dihydrid]

In einem Kolben werden 4.554 g (120 mmol) LiAlH₄ in 80 ml Diethylether gelöst. Unter Kühlung mit flüssigem Stickstoff werden in einem zweiten Kolben 5,334 g (40 mmol) wasserfreies Aluminiumtrichlorid in 80 ml Diethylether gelöst und dann bei Raumtemperatur der LiAlH₄-Lösung zügig zugetropft. Dabei fällt LiCl aus, und die Lösung wird trüb.

Der gebildeten Suspension werden unter Kühlung (0°C) langsam 11,859 g (160 mmol) tert.-Butanol zugetropft, wobei eine starke Wasserstoffentwicklung zu beobachten ist. Anschließend wird die Reaktionslösung noch 2 stunden bei Raumtemperatur gerührt. Das entstandene LiCl wird abgefrittet und das Lösungsmittel im Vakuum abgezogen. Der weiße Rückstand wird über Sublimation mittels eines mit Aceton/Trockeneis gekühlten Sublimationsrohres bei Raumtemperatur im Vakuum (1 mbar) gereinigt.

Ausbeute: 15,2 g (93 % der Theorie)

### Beispiel 2

### Präparation des Katalysators

Die Präparation des Katalysators erfolgt in einem "hot wall"-Reaktor. Das zu beschichtende Pulver befindet sich in einem Glasrohr, das mit der einen Seite mit der Precursorvorlage, mit der anderen an einer Rotoreinheit angeschlossen ist. Durch die Rotation des Rohres um seine Längsachse wird eine bessere Durchmischung und gleichmäßige Beschichtung des Pulvers erreicht. Das Heizen des Pulvers erfolgt mit einem Röhrenofen, in dem sich das Reaktorrohr befindet. Um den Precursor in die Gasphase zu überführen, wird im System Vakuum angelegt. Wegen der guten Flüchtigkeit des Precursors ist ein Erwärmen der Vorlage nicht erforderlich. Um einen kontinuierlichen Massentransport zu erreichen, wird der CVD-Prozeß unter dynamischen Vakuumbedingungen durchgeführt. Die flüchtigen Zersetzungsprodukte werden vor der Vakuumpumpe in einer Kühlfalle ausgefroren.

Ansatz:
4,50 g NiO/TiO₂ (Ni-Anteil 30 Gew.-%)
2,98 g [H₂AlOC₄H₉]
Umsetzung bei 400 °C
Ausbeute: 5,19 g Ni/Träger

Naßchemische Elementaranalyse:
- Ni:: 31,77 Gew.-%
- Al:: 1,49 Gew.-%

Die mittels Röntgendiffraktometrie ermittelten Kristallgrößen für NiO und Ni sind für unterschiedliche Katalysatoren in der nachstehenden Tabelle angegeben.

**Tabelle 1**

| **Volumengemittelte Teilchengröße, bestimmt über die Halbwertsbreite der Reflexe** | | |
|---|---|---|
| Ausgangspulver <D>ᵥ ^{NiO}/nM | Kat.-Probe/Temperatur/°C <D>ᵥ ^{NiO}/nm // <D>ᵥ ^{Ni}/nm | |
| *nano*-NiO 4,2 nm | Ni20Al/500 °C ------// 30 nm | |
| *nano-*NiO 4,2 nm | Ni30Al/450 °C 4,0 nm // 26 nm | |
| *polykristallines* NiO >500 nm | Ni33Al/500 °C 405 nm // 20 nm | |
| Ausgangspulver <D>ᵥ ^{NiO}/nm | Kat.-Probe/Temperatur/°C <D>ᵥ ^{NiO}/nm // <D>ᵥ ^{Ni}/nm | |
| NiO auf Träger/TiO₂ 10 nm | Ni38Al/500 °C ------// 20 nm | |
| NiO auf Träger/TiO₂ 10 nm | Ni43Al/400 °C ------// 12 nm | |

Die erfindungsgemäßen Katalysatoren sind nicht pyrophor und können an der Luft gehandhabt werden. Eine Aktivierung vor den Hydrierungsversuchen ist nicht notwendig.

### Beispiel 3

### Hydrierung von Dinitrotoluol

In einem 300 ml fassenden Rührautoklaven wurden 220 ml n-Butanol und 0,8 g des Katalysators aus Beispiel 2 vorgelegt. Es wurde bei 80 °C (bzw. 120 °C) für sechs Stunden eine Aktivierung unter 25 bar Wasserstoff durchgeführt.

Sodann wurden 20 g 2,4-Dinitrotoluol eingetragen und bei 80 °C und 25 bar Wasserstoff hydriert. Durch den Wasserstoffverbrauch wurde ein Druckverlust beobachtet, und Wasserstoff wurde jeweils nachgepreßt. Die Aktivität des Katalysators wurde anhand des pro Minute nachzupressenden Drucks bestimmt.

Die Aktivität erfindungsgemäßer Katalysatoren ist in der nachstehenden Tabelle mit der Aktivität bekannter Katalysatoren verglichen. Zudem wurden nach Lagerung an Luft aktivierte Katalysatoren untersucht. Die Ergebnisse sind in der nachstehenden Tabelle 2 zusammengefaßt.

**Tabelle 2**

| Katalysator | Aktivität (bar/min) |
|---|---|
| Ni/TiO₂ frisch reduziert, Luftausschluß (Vergleich) | 1,0 |
| Ni/TiO₂ passiviert (Vergleich) | 0,0 |
| Ni/TiO₂ passiviert, aktiviert 120 °C | 0,0 |
| Ni/AlOₓ/TiO₂ (400 °C) 3 Wochen an Luft | 0,17 |
| Ni/AlOₓ/TiO₂ (400 °C) 3 Wochen an Luft, aktiviert 80 °C, 6 h | 1,0 |
| Ni/Al/Oₓ/TiO₂ (500 °C) 3 Wochen an Luft | 0,22 |
| Ni/AlOₓ/TiO₂ (500 °C) 3 Wochen an Luft, aktiviert 80 °C, 6 h | 0,44 |

Bei den letzten vier Katalysatoren handelt es sich um erfindungsgemäße Katalysatoren. Die Temperaturangabe in Klammern bezieht sich auf die Umsetzungstemperatur bei der Herstellung des Katalysators. Die Herstellung erfolgte gemäß Beispiel 2.

Aus den Ergebnissen der Tabelle 2 geht hervor, daß sich die erfindungsgemäßen Katalysatoren selbst nach längerer Lagerung an Luft gut aktivieren lassen.

## Patentansprüche

1. Katalysator, herstellbar durch Reduktion eines, auf einem oxidischen Träger, ausgewählt aus TiO₂, ZrO₂ oder Gemischen davon, vorliegenden Ni-Precursors durch Umsetzung mit mindestens einer Verbindung der allgemeinen Formel (I)
HₙM (OR)₃₋ₙ (I)
mit
M Ga oder Al,
R CR'₃ oder SiR'₃ mit R' C₁₋₂₀-Alkyl,
n 1 oder 2
oder Dimeren oder Oligomeren davon.

2. Katalysator nach Anspruch 1, **dadurch gekennzeichnet, daß** die Verbindung der allgemeinen Formel (I) H₂Al(OCR'₃) mit R'C₁₋₃-Alkyl oder ein Dimer davon ist.

3. Katalysator nach einem der Ansprüche oder 2, **dadurch gekennzeichnet, daß** ein Oxid und/oder Hydroxid von Nickel bei der Umsetzung als Feststoff und die Verbindung der allgemeinen Formel (I) oder das Dimer oder Oligomer davon gasförmig eingesetzt werden.

4. Katalysator nach einem der Ansprüche 1 bis 3, **dadurch gekennzeichnet, daß** die Kristallitgröße des hydrieraktiven Metalls im fertigen Katalysator 5 bis 30 nm beträgt.

5. Verfahren zur Herstellung eines Katalysators durch Reduktion eines auf einem oxidischen Träger, ausgewählt aus TiO₂, ZrO₂ oder Gemischen davon, vorliegenden-Ni-Precursors durch Umsetzung mit mindestens einer Verbindung der allgemeinen Formel (I)
HₙM(OR)₃₋ₙ (I)
mit
M Ga oder Al,
R CR'₃ oder SiR'₃ mit R'C₁₋₂₀-Alkyl,
n 1 oder 2
oder Dimeren oder Oligomeren davon.

6. Verwendung eines Katalysators gemäß einem der' Ansprüche 1 bis 4 zur Hydrierung, Dehydrierung oder Isomerisierung von, gegebenenfalls substituierten, Kohlenwasserstoffen.

7. Verfahren zur Herstellung von aromatischen Verbindungen, die Aminogruppen aufweisen, durch Hydrierung entsprechender Nitrogruppen aufweisender aromatischer Verbindungen in Gegenwart eines Katalysators nach einem der Ansprüche 1 bis 4.

## Claims

1. Catalyst able to be produced by the reduction of a Ni precursor present on a support in form of an oxide selected from TiO₂, ZrO₂ or mixtures thereof by reaction with at least one component according to the general formula (I)
HₙM (OR)₃₋ₙ (I)
with
M Ga or Al,
R CR'₃ or SiR'₃ with R'C₁₋₂₀-alkyl,
n 1 or 2
or dimers or oligomers thereof.

2. Catalyst according to claim 1, **characterized in that** the component according to the general formula (I) is H₂Al(OCR'₃), R' being C₁₋₃-alkyl, or a dimer thereof.

3. Catalyst according to one of claims 1 or 2, **characterized in that** during the reaction, an oxide and/or hydroxide of Nickel is used in solid state and a compound according to the general formula (I) or the dimer or oligomer thereof is used in gaseous state.

4. Catalyst according to one of claims 1 or 3, **characterized in that** the size of the crystallites of the metal active in hydrogenation in the final catalyst is between 5 and 30 nm.

5. Process for the production of a catalyst by reduction of a precursor present on a support in form of an oxide selected from TiO₂, ZrO₂ or mixtures thereof by reaction with at least one component according to the general formula (I)
HₙM (OR)₃₋ₙ (I)
with
M Ga or Al,
R CR'₃ or SiR'₃ with R'C₁₋₂₀-alkyl,
n 1 or 2
or dimers or oligomers thereof.

6. Use of a catalyst according to one of claims 1 to 4 for the hydrogenation, the dehydrogenation or the isomerisation of hydrocarbons, if appropriate of substituted hydrocarbons.

7. Process for the production of aromatic compounds comprising amino groups by hydrogenation of aromatic compounds having corresponding nitro groups in presence of a catalyst according to one of claims 1 to 4.

## Revendications

1. Catalyseur pouvant être produit par la réduction d'un précurseur Ni présent sur un support sous forme d'un oxyde sélectionné entre TiO₂, ZrO₂ ou de mélanges de ceux-ci, en le faisant réagir avec au moins un composé obéissant à la formule générale (I)
HₙM(OR)₃₋ₙ (I)
avec
M Ga ou Al,
R CR'₃ ou SiR'₃ avec R' C₁₋₂₀-alkyle,
n 1 ou 2
ou des dimères ou des oligomères de celui-ci.

2. Catalyseur selon la revendication 1, **caractérisé en ce que** le composé obéissant à la formule générale (I) est H₂Al(OCR'₃), R' étant C₁₋₃-alkyle, ou un dimère de celui-ci.

3. Catalyseur selon l'une des revendications 1 ou 2, **caractérisé en ce que** lors de la réaction, un oxyde et/ou hydroxyde de Nickel est utilisé sous forme solide et un composé obéissant à la formule générale (I) ou le dimère ou l'oligomère de celui-ci est utilisé sous forme gazeuse.

4. Catalyseur selon l'une des revendications 1 à 3, **caractérisé en ce que** la taille de cristallites du métal actif pour l'hydrogénation dans le catalyseur final est entre 5 et 30 nm.

5. Procédé pour la production d'un catalyseur par la réduction d'un précurseur présent sur un support sous forme d'un oxyde sélectionné entre TiO₂, ZrO₂ ou de mélanges de ceux-ci, en le faisant réagir avec au moins un composé obéissant à la formule générale (I)
HₙM (OR)₃₋ₙ (I)
avec
M Ga ou Al,
R CR'₃ ou SiR'₃ avec R' C₁₋₂₀-alkyle,
n 1 ou 2
ou des dimères ou des oligomères de celui-ci.

6. Utilisation d'un catalyseur selon l'une des revendications 1 à 4 pour l'hydrogénation, la déshydrogénation ou l'isomérisation d'hydrocarbures, le cas échéant d'hydrocarbures substitués.

7. Procédé pour la production de composés aromatiques comportant des groupes aminés par l'hydrogénation de composés aromatiques ayant des groupes nitro correspondants en présence d'un catalyseur selon l'une des revendications 1 à 4.
